# EUROPEAN PATENT SPECIFICATION

(11) **EP 2 780 306 B1**
(45) Date of publication and mention of the grant of the patent: **09.08.2017**
(21) Application number: 12850051.9
(22) Date of filing: 15.11.2012
(51) Int. Cl.: C07C 7/20, B29C 45/18

(54) **LIQUID MOLDING RESIN WITH NONSWELLING MICA**
FLÜSSIGE FORMMASSE MIT NICHTQUELLENDER GLIMMERERDE
RÉSINE DE MOULAGE LIQUIDE AYANT DU MICA NE GONFLANT PAS

(30) Priority: 18.11.2011 US 201113299477
(43) Date of publication of application: 24.09.2014
(73) Proprietor: Metton America, Inc., LaPorte, TX 77571 (US)
(72) Inventor: BABA, Hiromitsu, Chiba 290-8503 (JP); KATO, Takeshi, Tokyo 104-8502 (JP); MINAMI, Hiroyuki, Chiba 290-8503 (JP); ABE, Masanori, Laporte, TX 77571 (US); YOKOO, Yusuke, Laporte, TX 77571 (US); WEST, Beau, Jeremy, Laporte, TX 77571 (US)
(74) Representative: Copsey, Timothy Graham
(86) International application number: PCT/US2012/065157
(87) International publication number: WO 2013/074719

(56) References cited:
- US-A- 4 400 340
- US-A1- 2010 009 158
- US-A1- 2011 245 417
- US-A2- 2008 097 008
- US-B1- 6 326 428
- US-E- R E35 717
- Jia-Qian Jiang ET AL: "Comparison of modified montmorillonite adsorbents. Part I: Preparation, characterization and phenol adsorption", Chemosphere, 1 May 2002 (2002-05-01), pages 711-716, XP055213803, England DOI: 10.1016/S0045-6535(02)00011-5 Retrieved from the Internet: URL:http://www.ncbi.nlm.nih.gov/pubmed/120 79066

## Description

### BACKGROUND OF THE INVENTION

### Field of the Invention

The present invention relates to a liquid molding resin and, more particularly, to a liquid resin for use in reaction injection molding.

### Description of the Related Art

A process is known for forming molded articles using thermoset polymers such as, for example, lightly cross-linked thermoset polymers based on polydicyclopentadiene (poly-DCPD or PDCPD). In such a process, two (or more) DCPD liquid streams are mixed together at a temperature near room temperature and are injected into a closed mold. One of the streams contains an activator and the other stream contains a catalyst. When the streams are mixed together, an exothermic reaction occurs (a polymerization crosslinking reaction) and a fully polymerized product is created.

There are two principal problems exhibited by PDCPD molded resin products. The first problem is that stiffness of the molded resin is not sufficient for some heavy duty applications. In order to make up for the insufficient stiffness, a higher thickness or a reinforcement attachment to the part was required in some cases.

The second problem is that coefficient of thermal expansion (CTE) of PDCPD molded resin products is not so low as other resins. A lower CTE is desirable, especially in the case of large and/or thick parts, to improve dimensional stability.

Addition of a filler was widely known to improve these problems for plastics. This technique was also investigated for possible application to resins such as PDCPD. An example of this technique is disclosed in US Patent No.4400340. A filler was used to increase the polymer's flexible modulus. Glass, wollastonite, carbon black, talc, calcium carbonate, and mica were exemplified as possible fillers.

Mica is one of the useful fillers for plastics. Mica is classified as one of two kinds of mica, which are swelling mica and non-swelling mica.

Japanese Laid-open Patent Application No. 2001-302888 discloses a technique of adding layer silicate containing organic onium ion into PDCPD molded resin as a filler. Smectite clay such as montmorillonite, vermiculite, and halloysite, and swelling mica were exemplified as layer silicate, and swelling mica especially was specified as a favorable material. This swelling mica originally contained a metal cation in the layered structure, and was ion-exchanged with organic onium for organification. This organified swelling mica had improved affinity with olefin metathesis polymer, and it enabled good dispersion in the polymer matrix. This technique was also disclosed in Japanese Laid-open Patent Application No. 2004-250635.

However, swelling mica is generally synthesized artificially, and is generally more expensive than nonswelling mica, which is natural product. This technique also required the additional procedure for ion exchange of swelling mica as an extra processing of material, which was a costly procedure.

Swelling mica was also known to delaminate to a thin-layer by absorbing solvent (swelling), and to have a high aspect ratio. However, the average particle size was very small as a range of 0.01 micro m to 3 micro m inherently (Section [0049] of Japanese Laid-open Patent Application No. 2001-302888), so that it had a problem that the viscosity increased significantly by its strong thixotropic behavior when adding a high content of swelling mica into a reaction monomer liquid in order to obtain a higher reinforcement effect. This viscosity increase is a critical drawback for the reaction injection molding method because it causes a bad mixing problem of the material at the mixing-head, and results in problems such as bad reaction, low stiffness, high residual monomer, poor quality of molded parts, etc. Therefore, the amount of swelling mica in the reaction monomer liquid has a limitation, which became insufficient to achieve the reinforcement effect.

As described above, the conventional method using mica had problems of inadequately high stiffness and excessive increase of reaction monomer liquid viscosity. The present inventors also observed poor reactivity during injection molding which was due to hindrance of the mica.

US 4,400,240 relates to a method of making a thermoset polydicyclopentadiene by first combining a plurality of reactant streams. US 6,326,428 relates to a mica reinforced reaction injection molded polyurethane/ polyurea. Jia-Qian Jiang et al., Chemosphere 47, 2002, 711 - 716 relates to a comparison of modified montmorillonite adsorbents.

### SUMMARY OF THE INVENTION

The present invention is directed to liquid molding resin components and a liquid molding process that can produce molded resin products having a high stiffness and a low coefficient of thermal expansion (CTE) of molded resin products with an low anisotropic property.

The inventors have found that a liquid resin component containing nonswelling mica having specific properties showed excellent fluidity and dispersion stability, which can result in an improved molded resin product.

Accordingly, one aspect of the present invention is directed to a liquid molding resin component for use in a reaction injection molding process, the liquid molding resin component comprising: a liquid resin reaction monomer comprising metathesis polymerizable cycloolefin; and nonswelling mica, and wherein the nonswelling mica have an average particle size in a range of 35 micro m to 500 micro m and have a bulk density in a range of 0.10 g/ml to 0.27 g/ml..

According to yet another aspect, the present invention is directed to a liquid resin component system for use in a reaction injection molding process, the component system comprising a plurality of liquid resin components, wherein each of the liquid resin components includes a reaction monomer comprising metathesis polymerizable cycloolefin, wherein at least one of the liquid resin components includes a catalyst component of a metathesis polymerization catalyst system and at least one of the liquid resin components includes an activator component of a metathesis polymerization catalyst system, and wherein at least one of the liquid resin components includes nonswelling mica having an average particle size in a range of 35 micro m to 500 micro m and having a bulk density in a range of 0.10 g/ml to 0.27 g/ml.

According to still another aspect, the present invention is directed to a method of making a liquid resin component for use in an injection molding process, the method comprising the steps of: providing a liquid resin reaction monomer wherein the reaction monomer is metathesis polymerizable cycloolefin; and adding nonswelling mica to the liquid resin reaction monomer, wherein the nonswelling mica have an average particle size in a range of 35 micro m to 500 micro m and have a bulk density in a range of 0.10 g/ml to 0.27 g/ml.

Yet another aspect of the present invention is directed to a method of making a molded resin product, the method comprising the steps of providing a plurality of liquid resin components that each include a reaction monomer that is metathesis polymerizable cycloolefin, wherein the reaction monomer in each of the plurality of liquid resin components is the same reaction monomer, wherein at least one of the liquid resin components includes a metathesis catalyst but not an activator, at least one of the liquid resin components includes a metathesis activator but not a metathesis catalyst, and at least one of the liquid resin components includes nonswelling mica; mixing the liquid resin components; and injecting the mixed liquid resin components into a mold at a predetermined temperature, wherein the nonswelling mica have an average particle size in a range of 35 micro m to 500 micro m and have a bulk density in a range of 0.10 g/ml to 0.27 g/ml.

These and other aspects of the present invention will be described in further detail below, with reference to the accompanying drawings.

### BRIEF DESCRIPTION OF THE DRAWINGS

FIG. 1 shows a system for making a molded resin product according to a preferred embodiment of the present invention.
FIG. 2 shows a process for making a molded resin product according to a preferred embodiment of the present invention.

### DETAILED DESCRIPTION OF THE PREFERRED EMBODIMENTS

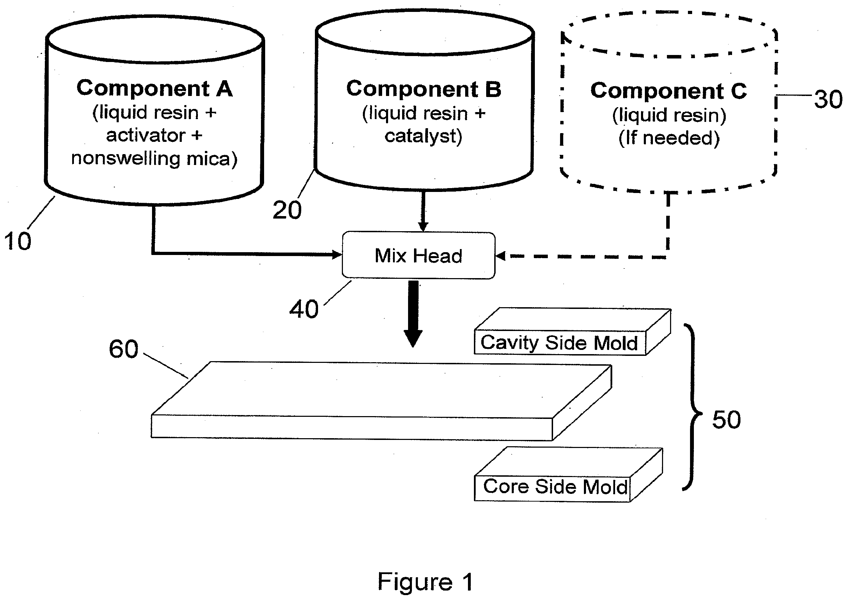

As shown in FIG. 1, a system for making a molded resin product according to a preferred embodiment of the invention includes a reservoir 10 (containing Component A), a reservoir 20 (containing Component B), a mix head 40, and a mold 50. Optionally, a reservoir 30 (containing Component C) may also be included.

Each of the reservoirs 10 and 20 (and reservoir 30, if used) includes a liquid resin component. The liquid resin component is a reaction monomer of a thermoset polymer, preferably a crosslinked olefinic thermoset polymer. Most preferably, the liquid resin components are based on reaction monomer comprising metathesis polymerizable cycloolefin. The metathesis polymerizable cycloolefin is preferably a compound having at least one norbornene skeltone
per molecule. The preferred examples of metathesis polymerizable cycloolefin are dicyclopentadiene, tricyclopentadiene, cyclopentadiene-methyl cyclopentadiene co-dimer, 5-ethylidenenorbornene, norbornene, norbornadiene, methyl norbornene, ethyl norbornene 5-cyclohexenylnorbornene, 1,4,5,8-dimethano-1,4,4a,5,6,7,8,8a-octahydronaphthalene, 1,4-methano-1,4,4a,5,6,7,8,8a-octahydronaphthalene, 6-ethylidene-1,4,5,8-dimethano-1,4,4a,5,6,7,8,8a-octahydronaphthalene, 6-ethylidene-1,4-methano-1,4,4a,5,6,7,8,8a-octahydronaphthalene, 1,4,5,8-dimethano-1,4,4a,5,8,8a-hexahydronaphthalene, ethylene-bis(5-norbornene), 5-methoxycarbonylnorbornene, 5-(2-ethylhexyloxy)carbonyl-5-methylnorbornene, 5phenyloxymethylnorbornene, 5-cyanonorbornene, 6-cyano-1,4,5,8-dimethano-1,4,4a,5,6,7,8,8a-octahydronaphthalene, N-butyl nadic acid imide, and 5-chloronorbornene.

These compounds may be used as the sole reaction monomer or in a mixture with other reaction monomers. A preferred embodiment uses a cycloolefin mixture containing 50 mol % or more, and more preferably 70 mol % or more, of dicyclopentadiene based on the total of cycloolefins. The most preferable embodiment uses a cycloolefin mixture that contains 80 mol % or more of dicyclopentadiene based on the total of cycloolefins.

At least one of the liquid resin components (e.g., Component A) includes an activator. The activator is preferably an organometallic compound, such as an alkylated metal of groups 1 to 3 of the periodic table of elements. For example, the activator may be an alkylaluminum compound such as triethyl aluminum, tributyl aluminum, trioctyl aluminum, or an alkyl aluminum halide compound such as diethyl aluminum chloride, ethyl aluminum dichloride, and dioctyl aluminum iodide, or a tetraalkyl tin compound such as tetrabutyl tin. Component A is prepared by dissolving the organometallic compound used as an activator into metathesis polymerizable cycloolefin.

When an alkyl aluminum is used as the activator, the weight ratio of the aluminum compound based on the total amount of metathesis polymerizable cycloolefin, or the mixture of reaction monomers, in Component A is preferably within a range of about 1 : 10 to about 1 : 1,000, and more preferably within a range of about 1 : 40 to about 1 : 200.

At least one of the other liquid resin components (e.g., Component B) includes a catalyst. The catalyst is preferably halide, oxihalide, oxide, ammonium salt of metals such as tungsten, rhenium, tantalum, molybdenum, or carbene complex of metals such as ruthenium and osmium. From the standpoint of reactivity, tungsten compounds are especially preferred. Among tungsten compounds, preferred examples of a catalyst include tungsten hexahalides such as tungsten hexachloride and tungsten oxyhalides such as tungsten oxychloride, Further, an organic ammonium salt of tungstic acid may also be used.

When a tungsten compound is used as the catalyst, the volume ratio of the tungsten compound based on the total amount of metathesis polymerizable cycloolefin (or the mixture of reaction monomers) in Component B is preferably within a range of about 1 : 5 to 1 : 500 and, more preferably, within a range of about 1 : 20 to 1 : 100.

If a tungsten compound such as those mentioned above is added directly to the metathesis polymerizable cycloolefin, cationic polymerization is immediately initiated. Therefore, when Component B is prepared using such a tungsten compound, the catalyst has to be deactivated in advance. More specifically, it is preferred that these tungsten compounds are prepared for use by suspending the compound in an appropriate inactive solvent such as, for example, benzene, toluene, and chlorobenzene, and adding a small amount of an alcohol-based compound and/or a phenol-based compound to the inactive solvent. Further, it is preferable that about 1 - 5 mol of Lewis base or a chelating agent be added to 1 mol of the tungsten compound in order to prevent the cationic polymerization. Examples of suitable additives include acetyl acetone, alkyl esters of alkyl acetate, tetrahydrofuran, and benzonitrile. The amount of chelating agent or the like can be appropriately selected according to the amount of the catalyst.

Further, with the above-described preferred composition, the polymerization reaction proceeds rapidly and may therefore be completed before the liquid resin flows in a sufficient amount into the mold. Accordingly, it is preferable to use a regulating agent to regulate the polymerization activity. A Lewis base is preferably used as such a regulating agent, and ethers, esters, and nitriles are especially preferred. Specific examples of preferred compounds for uses as regulating agents include ethyl benzoate, butyl ether, and diglyme. These regulating agents are preferably used by adding the compound to Component A containing an organometallic compound as an activator, and the amount of the regulating agent is appropriately selected according to the amount of the catalyst being used.

The optional Component C is preferably also a metathesis polymerizable cycloolefin liquid resin containing neither an activator nor a catalyst. Nonswelling mica can be added to Component C instead of, or in addition to, adding them to Component A and/or Component B.

Each of Component A, Component B, and (if used) Component C may also include other additives to improve or maintain the properties of the molded resin. For example, such additives may serve as fillers, pigments, antioxidants, photostabilizers, flame retardants, foaming agents, mold releasing agents, deodorants, plasticizers, lubricants, antistatic agents, or polymer conditioners. These additives may be used alone or in combination.

Other polymer components may also be added to the liquid resin components. For example, elastomers are often used as polymer additives to increase the impact strength of the molded resin and to control the viscosity of the liquid resin. Specific examples of preferred elastomers include styrene-butadiene-styrene block rubber, styrene-isoprene-diene-terpolymer, nitrile rubber, a styrene block rubber, polybutadiene, polyisoprene, butyl rubber, ethylene-propylene-diene- terpolymer, and nitrile rubber.

At least one of the liquid resin components also includes nonswelling mica. The "swelling" means when layered silicate such as swelling mica are contacted with water, alcohol, or organic solvent, the distance between each layer of the layered silicate is enlarged by intercalation of solvent molecules between the layers. Some swelling mica exhibits a phenomenon in water, alcohol, or organic solvent wherein a multi-layered structure of the swelling mica is dissociated to a single independent layer. Thus, "nonswelling mica" means types of mica that do not show such "swelling" when the mica is contacted with water, alcohol, or organic solvent.

Nonswelling mica as used in this invention can be obtained from materials widely used as a filler or for reinforcement in the plastics industry. Preferred examples of nonswelling mica are natural product mica such as muscovite, phlogopite, annite, biotite, and sericite. Also, artificial product mica such as fluoro-phlogopite [KMg₃(AlSi₃O₁₀)F₂], potassium tetrasilisic mica [KMg_{2.5}(Si₄O₁₀)F₂] may be used as nonswelling mica in the present invention. A further example is calcinated mica, which is synthesized from natural product mica by calcination wherein a hydroxide component in the mica structure is removed. The most favorable examples of nonswelling mica are muscovite and phlogopite.

Nonswelling mica as used in this invention preferably have a specific bulk density. The bulk density is preferably in a range of about 0.10 g/ml to about 0.27 g/ml, and more preferably in a range of about 0.14 g/ml to about 0.25 g/ml. If the bulk density is larger than about 0.27 g/ml, the dispersion of the nonswelling mica in the reaction monomer liquid deteriorates and a lump of the nonswelling mica may result in the liquid. It also causes poor stability of dispersion of the nonswelling mica, and the nonswelling mica settle out in the liquid quickly during the reaction injection molding procedure. This fast settling of the particles in the liquid may cause a clog at a pipe line, tank, filter, or injection nozzle of the injection machine, where the liquid flows. On the other hand, if the bulk density is smaller than about 0.10 g/ml, the viscosity of the reaction monomer liquid containing the nonswelling mica is excessively increased and it becomes difficult to mix it, and in some cases it causes bad reactivity during the injection molding.

Nonswelling mica as used in this invention preferably has a specific average particle size. The average particle size is preferably in a range of about 35 micro m to about 500 micro m, more preferably in a range of about 37 micro m to about 300 micro m, and most preferably in a range of about 40 micro m to about 200 micro m. If the average particle size is smaller than about 35 micro m, the modulus and strength of a molded product are not adequate, and in some cases bad reactivity results during the injection molding. On the other hand, if the average particle size is larger than about 500 micro m, the molded product becomes worse in the impact strength, the surface smoothness, and the appearance. It also may cause a clog at a pipe line, tank, filter, or injection nozzle of the injection machine, where the liquid flows.

Nonswelling mica generally comprises flat-shape particles. Therefore the average particle size means an average of the maximum diameters of the flat particles. It is also generally defined as a particle diameter at 50% of accumulated volume (or accumulated weight) (D50) on a cumulative distribution curve of particle diameter. The average particle size can be generally decided by laser diffractometry, a centrifugal sedimentation method, a sieve method, and so on.

It is not clear why the nonswelling mica with a small average particle size inhibits the reactivity of reaction monomer liquids during the injection molding. However, the inventors speculated as follows. It is generally known that the nonswelling mica has a weak negative charge on the particle surface. The nonswelling mica with a smaller average particle size has a larger surface area in the reaction monomer liquid, so that this negative charge in total is much higher than for nonswelling mica with a larger average particle size. This negative charge may affect the reactivity and cause hindrance.

Nonswelling mica as used in this invention preferably has a specific average aspect ratio. The average aspect ratio is preferably in a range of about 10 to about 200, more preferably in a range of about 15 to about 160, and most preferably in a range of about 20 to about 120. If the average aspect ratio is smaller than about 10, the modulus, strength and/or dimensional stability of a molded product are not adequate. On the other hand, if the average aspect ratio is larger than about 200, it may cause a problem of fast settling of the particles in the liquid, and may cause a clog at a pipe line, tank, filter, or injection nozzle of the injection machine, where the liquid flows. The average aspect ratio of nonswelling mica as used in this invention means an average of the ratio between the length of the major axis and the thickness of the flat particles. Furthermore, if an average particle size is outside of the above-mentioned preferred range, even though an average aspect ratio is within the above-mentioned preferred range, stiffness and impact strength of the molded product are not adequate because of increased viscosity of the reaction monomer liquid or the reaction hindrance during the injection molding.

Nonswelling mica can be added to any one, any two, or all of Component A, Component B, and/or Component C. Preferably nonswelling mica may be added into component C.

There is no specific limitation on the amount of nonswelling mica that may be added to Component A, Component B, and/or Component C, provided that the nonswelling mica can be uniformly dispersed in the liquid resin. However, the total amount of the nonswelling mica in all the liquid resin components is preferably within the range from about 6 to about 50 parts by weight of the total amount of liquid resin used in the molding process (that is, the liquid resin after mixing of the components). The total amount of nonswelling mica is more preferably in the range of about 8 to about 40 parts by weight of the total amount of liquid resin, furthermore preferably in the range of about 10 to about 30 parts by weight of the total amount of liquid resin. If the amount of nonswelling mica is less than about 6 parts by weight, the effects usually provided by the nonswelling mica, such as improved stiffness and dimensional stability in the molded resin, will not be obtained. On the other hand, if the amount of nonswelling mica is greater than about 50 parts by weight, the number of nonswelling mica in the molded resin will be too great and the mechanical properties will be adversely affected. It also causes a problem of fast settling of the particles in the liquid, and may cause a clog at pipe line, tank, filter, or injection nozzle of the injection machine, where the liquid flows.

The nonswelling mica used in this invention does not need any treatment before use, but it can be treated with a surface treatment agent before use. If the nonswelling mica is treated with a surface treatment agent before use, generally known surface treatment agents can be used. Preferable examples of a surface treatment agent are a silane coupling agent, titan coupling agent, higher fatty acid, higher fatty acid ester, higher fatty acid amide, and higher fatty acid salt. The most preferable example of a surface treatment agent is a silane coupling agent.

Preferable examples of silane coupling agents are vinyl methoxy silane, vinyl ethoxy silane, 2-(3,4-epoxycyclohexyl)ethyl trimethoxy silane, 3-glycidoxy propyl methyl dimethoxy silane, p-styryl methoxy silane, 3-methacryloxy dipropyl methyl diethoxy silane, 3-methacryloxy propyl trimethoxy silane, 3-methacryloxy propyl methyl diethoxy silane, 3-methacryloxy propyl triethoxy silane, 3-acryloxy propyl trimethoxy silane, and so on.

Any conventional surface treatment method can be applied, if the nonswelling mica is treated with a surface treatment agent. One preferable example of a surface treatment is as follows. A surface treatment agent such as a silane coupling agent is added into a solution such as water, alcohol, organic solvent, or a mixture of them, which may or may not include a pH adjuster such as acetic acid. The nonswelling mica is added into the prepared solution, and mixed uniformly. Then, the treated nonswelling mica is filtered from the liquid, and dried to remove solvent if necessary. Another preferable example of a surface treatment method is a spray method, wherein a surface treatment agent or a solution including a surface treatment agent is sprayed onto the nonswelling mica. The treated nonswelling mica may or may not be dried to remove any component such as solvent or excess surface treatment agent.

Fillers such as fibrous filler or particulate filler can be added together with the nonswelling mica used in this invention, if the properties of the molded product are not negatively affected. Preferable examples of fibrous fillers are glass fiber, wollastonite, potassium titanate, xonotlite, basic magnesium sulfate, aluminum borate, tetrapod-shaped zinc oxide, plaster fiber, phosphate fiber, alumina fiber, needle-like calcium carbonate, needle-like boehmite, and so on.

Preferable examples of particulate fillers are calcium carbonate, calcium silicate, calcium sulfate, aluminum hydroxide, magnesium hydroxide, titanium oxide, zinc oxide, barium titanate, silica, alumina, carbon black, graphite, antimony oxide, red phosphorus, hydrotalcite, and so on.

A component of the reaction monomer that includes the nonswelling mica preferably has a viscosity of 3.0 Pa·s or lower at 35 degrees C, and more preferably has a viscosity of 2.5 Pa·s or lower at 35 degrees C. The viscosity as used in this invention is measured by Sine-Wave Vibro Viscometers as described in the section below concerning the specific examples of the preferred embodiments.

A molded resin product according to this invention is obtained by reaction injection molding. Preferably, the above-described preferred compositions are injected into a mold, and the molded resin product is obtained by a polymerization reaction including cross-linkage reaction.

A method for manufacturing a molded resin product according to the present invention may use a conventional reaction injection molding (RIM) machine that is known in the related arts. As shown in Figure 1, when injection molding is to be carried out, each of the reservoirs 10, 20, and (if used) 30 provides a liquid stream to the mix head 40. The respective airtight storage containers may serve as the reservoirs, if there is appropriate provision for connection a pump or the like to transfer the liquid resin component to the mix head 40. The liquid resin components are preferably maintained at a temperature in the range of about 10 - 50 degree C. Homogeneous premixing is performed by mix head 40. After the liquid resin components are mixed together by mix head 40, they are injected into mold 50, which is set preferably at 10 - 120 degree C, more preferably at 30 - 100 degree C. An exothermic reaction takes place, which creates a fully polymerized molded resin product 60.

The pressure maintained within the mold during the molding reaction is preferably within a range of about 0 - 1 MPa, and more preferably within a range of about 0.02 - 0.5 MPa.

There is no limitation on the curing time of the molding reaction, and it may be selected according to the particular application, but it is preferably about 5 sec to about 30 min, and more preferably about 20 sec to about 10 min, after the injection is completed.

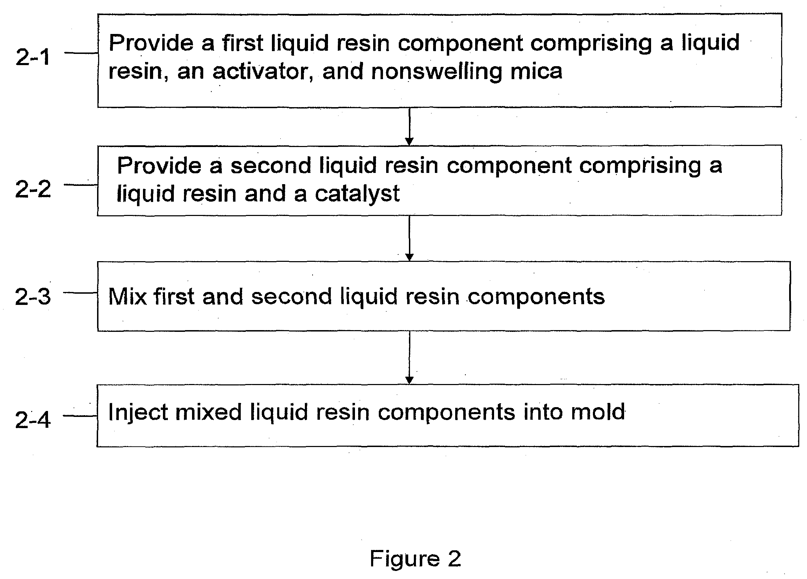

Fig. 2 is a flow diagram illustrating a process for making a molded resin product. In step 2-1, a first liquid resin component is provided that comprises a liquid resin reaction monomer, an activator, and nonswelling mica. In step 2-2, a second liquid resin component is provided that comprises a liquid resin reaction monomer and a catalyst. In step 2-3, the first and second liquid resin components are mixed together. As mentioned above, the resin components are preferably mixed homogeneously with a 1 : 1 ratio. In step 2-4, the mixed liquid resin is injected into a mold.

Although in the above description the nonswelling mica are included in the liquid resin component containing the activator, it should be apparent to those skilled in the art that the nonswelling mica could instead be included in the liquid resin component that contains the catalyst, or some nonswelling mica could be included in both liquid resin components. Further, those of ordinary skill in the field of thermoset resins will understand that a third resin component containing liquid resin and nonswelling mica and/or other additives may be used.

The molded resin product obtained by the above mentioned method preferably has both flexural strength and tensile strength in the flow direction and the lateral direction with respect to the injection flow, of 2.8 GPa or higher, fmore preferably 3.3 GPa or higher, and most preferably 3.8 GPa or higher. The flexural strength and tensile strength are measured by a method conforming to Japanese Industrial standards (JIS) K7171.

The molded resin product also preferably has a low anisotropic coefficient of thermal expansion (CTE) between the flow direction and the lateral direction with respect to the injection flow. The ratio of CTE in the flow direction (f) to that in the lateral direction (v) is preferably in a range between 0.80 and 1.25, more preferably in a range between 0.85 and 1.18, and most preferably in a range between 0.90 and 1.11. The coefficient of thermal expansion (CTE) is measured by a method conforming to Japanese Industrial standards (JIS) K7197.

The molded resin product also preferably has a notched izod impact strength in the flow direction (MD) and in the lateral direction(TD) with respect to the injection flow of 100 J/m or more. The notched izod impact strength is measured by a method conforming to Japanese Industrial standards (JIS) K7110.

As described above, a molded resin product according to the present invention has excellent properties of stiffness, dimensional stability, and impact strength, and also a very low anisotropic property as well. Therefore the molded article can be utilized in various applications such as plastic parts for an automobile, truck, tractor, or septic tank, in construction, and so on.

### SPECIFIC EXAMPLES OF PREFERRED EMBODIMENTS

Specific examples of preferred embodiments are provided below to further illustrate aspects of the present invention. Those skilled in the art will appreciate that there are numerous other variations that can be formulated based on the teachings of this specification, and the examples below are not intended to limit the scope of the present invention.

In the following examples, the mixed reaction monomer, activator, and catalyst are prepared as follows:

### (Preparation of mixed reaction monomer)

A total weight of 4 parts by weight of ethylene-propylene-ethylidene norbornene co-polymer rubber is dissolved into a solution consisting of 91 parts by weight of high-purity dicyclopentadiene (purity 99.7 wt.%) and 5 parts by weight of ethylidene norbornene (purity 99.5 wt.%).

### (Preparation of activator)

Trioctylaluminum and diglyme were mixed in a molar ratio of 100: 100.

### (Preparation of catalyst)

28 parts by weight of tungsten hexachloride was added to 60 parts by weight of dry toluene under a nitrogen atmosphere and then a solution consisting of 1.3 parts by weight of t-butanol and 1 parts by weight of toluene was added. The solution was purged with nitrogen overnight to remove hydrogen chloride gas formed by the reaction of tungsten hexachloride with t-butanol. Then a solution consisting of 18 parts by weight of nonylphenol was added to the above solution. The mixed solution was purged with nitrogen overnight to remove hydrogen chloride gas formed by the reaction of the tungsten complex with nonylphenol. Then, 14 parts by weight of acetylacetone was added. The solution was purged with nitrogen overnight to remove hydrogen chloride gas formed by the reaction of the tungsten complex with acetylacetone. The resulting solution was used as the catalyst for polymerization.

[Preparation of filler with silane coupling treatment by solution (wet method)]

3.2 parts by weight of methanol, 0.03 parts by weight of acetic acid, 0.03 parts by weight of a silane coupling agent, 3-methacryloxy propyl trimethoxy silane, were added to 3.2 parts by weight of ion exchange water, and then mixed at room temperature for 3 hours. 3 parts by weight of filler was added into the above solution, and then mixed at room temperature for 1 hour. The treated filler was filtrated, and then dried in air at room temperature overnight. Then, the filler was dried by oven at 120 degree C for 20 min.

[Preparation of filler with silane coupling treatment by spraying (dry method)]

0.03 parts by weight of a silane coupling agent, 3-methacryloxy propyl trimethoxy silane, was added to 3 parts by weight of filler by spraying, and then mixed at room temperature for 1 hour.

In the following examples, the following properties were measured as described below.
(1) The flexural strength and tensile strength were measured by a method conforming to Japanese Industrial standards (JIS) K7171.
(2) The coefficient of thermal expansion (CTE) was measured by a method conforming to Japanese Industrial standards (JIS) K7197.
(3) The notched izod impact strength was measured by a method conforming to Japanese Industrial standards (JIS) K7110.
(4) The viscosity was measured by Sine-Wave Vibro Viscometers (manufactured by A&D company limited, SV type viscometer) at 35 degrees C.

### Example 1

### (Preparation of Component A)

A total weight of 1.2 parts by weight of activator was mixed into 100 parts by weight of the mixed monomer.

### (Preparation of Component B)

A total weight of 2.2 parts by weight of the catalyst was mixed into 100 parts by weight of the mixed monomer.

### (Preparation of Component C)

A total weight of 30 parts by weight of muscovite mica MC-100 (manufactured by Hayashi-Kasei Co., Ltd.) was treated with a silane coupling agent by the above-described wet method, and then was added into 70 parts by weight of the mixed monomer, and then mixed at room temperature for 1 hour. The obtained solution had a viscosity of 0.55 Pa·s at 35 degrees C. The MC-100 has an average particle size of 150 micro m and an average aspect ratio of 25.

### (Evaluation of dispersion of Component C)

Dispersion of Component C was checked by leaving it to stand for 1 hour and then looking to see if there was any apparent lump or sedimentation of the nonswelling mica or added filler. If there was a lump or sedimentation, it could cause a clog at a pipe line, tank, filter, or injection nozzle of the injection machine, and also results fluctuation of non swelling mica concentration which was injected into the molded parts.

### (Molding)

Component A, Component B, and Component C were mixed by a mixing head with a weight ratio of 1 : 1 : 2 and were injected into a mold. A mold for plaques was used having a length of 250 mm, a width of 250 mm, and a thickness of 3 mm. The mold temperature was 90 degrees C on the cavity side and 50 degrees C on the core side. The pressure inside the mold was maintained at 0.05 MPa during the molding.

### (Evaluation)

The appearance of the molded plaques surface was observed, and the result are presented in Table 1.

The molded plaques were cut to appropriate sizes to measure mechanical properties such as specific gravity, impact strength, tensile strength, tensile modulus, flexural strength, and flexural modulus. The measurements of these mechanical properties are presented in Table 1. "f" represents data measured with a cut specimen parallel to the longitudinal direction of the mold , which is a flow direction with respect to the injection flow. "v" represents data measured with a cut specimen parallel to the lateral direction of the mold, which is a lateral direction with respect to the injection flow. If the ratio of f to v (f / v) or v to f (v / f) is close to 1, it means there is only a small difference between properties in the flow direction and the lateral direction with respect to the injection flow, in other words, there is low anisotropy.

### Example 2

Except for changing the filler to muscovite mica MICA-100 (manufactured by Wakita Kogyo Co., Ltd.) which was treated with a silane coupling agent by the above-described wet method, the mixture solution of reaction monomer and filler was obtained in the same way as example 1. The obtained solution had a viscosity of 0.8 Pa·s at 35 degrees C. After leaving Component C to stand for 1 hour, there was observed neither a lump nor sedimentation. The MICA-100 has an average particle size of 75 micro m and an average aspect ratio of 35.

The molded plaques were obtained in the same way as example 1. The physical properties were measured in the same way as example 1. The results are presented in Table 1.

### Example 3

Except for changing the filler to muscovite mica B-82 (manufactured by Yamaguchi mica Co., Ltd.) which was treated with a silane coupling agent by the above-described dry method, the mixture solution of reaction monomer and filler was obtained in the same way as example 1. The obtained solution had a viscosity of 0.6 Pa·s at 35 degrees C. After leaving Component C to stand for 1 hour, there was observed neither a lump nor sedimentation. The B-82 has an average particle size of 180 micro m and an average aspect ratio of 100.

The molded plaques were obtained in the same way as example 1. The physical properties were measured in the same way as example 1. The results are presented in Table 1.

### Example 4

Except for changing the filler to muscovite mica YM-41 S (manufactured by Yamaguchi mica Co., Ltd.) which was treated with a silane coupling agent by the above-described wet method, the mixture solution of reaction monomer and filler was obtained in the same way as example 1. The obtained solution had a viscosity of 1.0 Pa·s at 35 degrees C. After leaving Component C to stand for 1 hour, there was observed neither a lump nor sedimentation. The YM-41S has an average particle size of 47 micro m and an average aspect ratio of 85.

The molded plaques were obtained in the same way as example 1. The physical properties were measured in the same way as example 1. The results are presented in Table 1.

### Example 5

Except for changing the filler to muscovite mica MICA-100 (manufactured by Wakita Kogyo Co., Ltd.) without a silane coupling treatment, the mixture solution of reaction monomer and filler was obtained in the same way as example 1. After leaving Component C to stand for 1 hour, there was observed neither a lump nor sedimentation. The obtained solution had a viscosity of 1.0 Pa·s at 35 degrees C.

The molded plaques were obtained in the same way as example 1. The physical properties were measured in the same way as example 1. The results are presented in Table 1.

### Example 6

Except for changing the amount of filler to a total weight of 40 parts by weight of muscovite mica MICA-100 (manufactured by Wakita Kogyo Co., Ltd.) which was treated with a silane coupling agent by the above-described wet method, the mixture solution of reaction monomer and filler was obtained in the same way as example 1. After leaving Component C to stand for 1 hour, there was observed neither a lump nor sedimentation. The obtained solution had a viscosity of 2 Pa·s at 35 degrees C.

The molded plaques were obtained in the same way as example 1. The physical properties were measured in the same way as example 1. The results are presented in Table 1.

### Comparative Example 1

Except for not using component C, the molded plaques were obtained from component A and component B in the same way as Example 1. The physical properties were measured in the same way as example 1. The results are presented in Table 1.

### Comparative Example 2

Except for changing the filler to muscovite mica A-21 (manufactured by Yamaguchi mica Co., Ltd.) which was treated with a silane coupling agent by the above-described wet method, the mixture solution of reaction monomer and filler was obtained in the same way as example 1. The obtained solution had a viscosity of 3.1 Pa·s at 35 degrees C. After leaving Component C to stand for 1 hour, there was observed neither a lump nor sedimentation. The A-21 has an average particle size of 22 micro m and an average aspect ratio of 70.

The molding of plaques was tried in the same way as example 1, but molded plaques could not be obtained due to bad reactivity. Therefore, the physical properties could not be measured in the same way as example 1.

Then the amount of filler was decreased to a total weight of 5 parts by weight, and the mixture solution of the reaction monomer and the filler was obtained in the same way as example 1. The obtained solution had a viscosity of 0.16 Pa·s at 35 degrees C.

The molded plaques were obtained with the mixture solution having the decreased amount of filler in the same way as example 1. The physical properties were measured in the same way as example 1. The results are presented in Table 1.

### Comparative Example 3

Except for changing the filler to phlogopite mica W-40H (manufactured by Repco Inc.) which was treated with a silane coupling agent by the above-described wet method, the mixture solution of reaction monomer and filler was obtained in the same way as example 1. The obtained solution had a viscosity of 2.2 Pa·s at 35 degrees C. After leaving Component C to stand for 1 hour, there was observed neither a lump nor sedimentation. The W-40H has an average particle size of 33 micro m and an average aspect ratio of 45.

The molding of plaques was tried in the same way as example 1, but molded plaques could not be obtained due to bad reactivity. Therefore the physical properties could not be measured in the same way as example 1.

Then the amount of filler was decreased to a total weight of 10 parts by weight, and the mixture solution of the reaction monomer and the filler was obtained in the same way as example 1. The obtained solution had a viscosity of 2.0 Pa·s at 35 degrees C.

The molded plaques were obtained with the mixture solution having the decreased amount of filler in the same way as example 1. The physical properties were measured in the same way as example 1. The results are presented in Table 1.

### Comparative Example 4

Except for changing the filler to phlogopite mica S-150H (manufactured by Repco Inc.) which was treated with a silane coupling agent by the above-described dry method, the mixture solution of reaction monomer and filler was obtained in the same way as example 1. The obtained solution had a viscosity of 0.6 Pa·s at 35 degrees C. After leaving Component C to stand for 1 hour, there was observed a lump of the mica. The S-150H has an average particle size of 160 micro m and an average aspect ratio of 80.

The molding of plaques was tried in the same way as example 1, but molded plaques could not be obtained because the injection nozzle of the injection machine was clogged. Therefore the physical properties could not be measured in the same way as example 1.

### Comparative Example 5

Except for changing the filler to muscovite mica HR-90 (manufactured by Kish Company Inc.) which was treated with a silane coupling agent by the above-described wet method, the mixture solution of reaction monomer and filler was obtained in the same way as example 1. The obtained solution had a viscosity of 0.5 Pa·s at 35 degrees C. The HR-90 has an average particle size of 720 micro m.

The molding of plaques was tried in the same way as example 1, but molded plaques could not be obtained because the injection nozzle of the injection machine was clogged. Therefore, the physical properties could not be measured in the same way as example 1.

### Comparative Example 6

Except for changing the amount of filler to a total weight of 10 parts by weight, the mixture solution of the reaction monomer and the filler was obtained in the same way as example 1. After leaving Component C to stand for 1 hour, there was observed neither a lump nor sedimentation. The obtained solution had a viscosity of 0.22 Pa·s at 35 degrees C.

The molded plaques were obtained in the same way as example 1. The physical properties were measured in the same way as example 1. The results are presented in Table 1.

### Comparative Example 7

Except for changing the filler to talc PK-P (manufactured by Hayashi-Kasei Co., Ltd.) which was treated with a silane coupling agent by the above-described wet method, the mixture solution of the reaction monomer and the filler was obtained in the same way as example 1. The obtained solution had a viscosity of 3.2 Pa·s at 35 degrees C. After leaving Component C to stand for 1 hour, there was observed neither a lump nor sedimentation. The PK-P has an average particle size of 6.5 micro m.

The molded plaques were obtained in the same way as example 1. The physical properties were measured in the same way as example 1. The results are presented in Table 1.

### Comparative Example 8

Except for changing the filler to fibrous fiber, wollastonite Nyglos 8 (manufactured by NYCO Minerals Inc) which was treated with a silane coupling agent by the above-described wet method, the mixture solution of the reaction monomer and the filler was obtained in the same way as example 1. The obtained solution had a viscosity of 2.2 Pa·s at 35 degree C. After leaving Component C to stand for 1 hour, there was observed segmentation of the wollastonite. The Nyglos 8 has an average fiber length of 136 micro m and an average fiber diameter of 8 micro m.

The molded plaques were obtained in the same way as example 1. The physical properties were measured in the same way as example 1. The results are presented in Table 1.

The above-described results and the data in Table 1 illustrate that a molded resin product formed in accordance with the present disclosure has mechanical properties superior to those obtained using conventional fillers. Thus, it is clear that a molded resin product can be obtained practically that has a high stiffness and a low coefficient of thermal expansion (CTE). Further, a molded resin product can be obtained that has only a small difference of physical properties between the flow direction and the lateral direction with respect to the injection flow (*i.e.*, low anisotropy. Furthermore, a molded resin product can be obtained that has a pearlescent surface without extra processing or additional equipment.

While the invention has been described above by way of examples and preferred embodiments, those skilled in the art will recognize that there other variations of the above embodiments. The scope of the invention is not intended to be limited to the specific examples and embodiments presented above, but rather should be determined by reference to the claims appended hereto.

**Table 1 (part 1)**

| | Example 1 | Example 2 | Example 3 | Example 4 | Example 5 |
|---|---|---|---|---|---|
| Filler | MC-100 | MICA-100 | B-82 | YM-41 S | MICA-100 |
| Type of filler | Muscovite mica | Muscovite mica | Muscovite mica | Muscovite mica | Muscovite mica |
| Average particle size of filler (micro m) | 150 | 75 | 180 | 47 | 75 |
| Average aspect ratio of filler | 25 | 35 | 100 | 85 | 35 |
| Bulk density of filler (g/ml) | 0.25 | 0.26 | 0.23 | 0.17 | 0.26 |
| Viscosity of C Component (Pa·s) | 0.55 | 0.8 | 0.6 | 1.0 | 1.0 |
| Lump or segmentation of filler in C Component (Yes/No) | No | No | No | No | No |
| Content of filler in molded plaques (wt%) | 15 | 15 | 15 | 15 | 15 |
| Flexural Modulus f (GPa) | 3.89 | 4.3 | 3.72 | 4.43 | 4.02 |
| Flexural Modulus v (GPa) | 3.4 | 4.41 | 3.22 | 4.27 | 3.93 |
| v/f | 0.87 | 1.03 | 0.87 | 0.96 | 0.98 |
| Tensile Modulus f (GPa) | 3.35 | 3.6 | 2.95 | 3.91 | 3.61 |
| Tensile Modulus v (GPa) | 3.34 | 3.29 | 2.8 | 3.69 | 3.37 |
| v/f | 1.00 | 0.91 | 0.95 | 0.94 | 0.93 |
| CTE f (10⁻⁵/degree C) | 5.44 | 4.83 | 5.71 | 5 | 5.08 |
| CTE v (10⁻⁵/degree C) | 5.92 | 5.07 | 6.18 | 5.08 | 5.49 |
| f/v | 0.92 | 0.95 | 0.92 | 0.98 | 0.93 |
| Notched Izod Impact strength f (J/m) | 119 | 110 | 122 | 162 | 194 |
| Notched Izod Impact strength v (J/m) | 100 | 103 | 103 | 136 | 165 |
| v/f | 0.84 | 0.94 | 0.84 | 0.84 | 0.85 |
| Appearance of molded plaque surface | Pearlescent Yellow | Pearlescent yellow | Pearlescent yellow | Pearlescent yellow | Pearlescent yellow |

**Table 1 (part 2)**

| | Example 6 | Comparative Example 1 | Comparative Example 2 | Comparative Example 3 | Comparative Example 4 |
|---|---|---|---|---|---|
| Filler | MC-100 | None | A-21 | W-40H | S-150H |
| Type of filler | Muscovite mica | | Muscovite mica | Phlogopite mica | Phlogopite mica |
| Average particle size of filler (micro m) | 150 | | 22 | 33 | 160 |
| Average aspect ratio of filler | 25 | | 70 | 45 | 80 |
| Bulk density of filler (g/ml) | 0.25 | | 0.13 | 0.18 | 0.28 |
| Viscosity of C Component (Pa·s) | 2 | | 3.1 | 2.2 | 0.6 |
| Lump or segmentation of filler in C Component (Yes/No) | No | | No | No | Yes |
| Content of filler in molded plaques (wt%) | 20 | 0 | 5 | 10 | 15 |
| Flexural Modulus f (GPa) | 4.6 | 1.9 | 2.3 | 3.36 | N/A |
| Flexural Modulus v (GPa) | 4.2 | 1.9 | 2.3 | 3.3 | N/A |
| v/f | 0.91 | 1.00 | 1.00 | 0.98 | N/A |
| Tensile Modulus f (GPa) | 3.9 | 1.9 | 2.3 | 3.12 | N/A |
| Tensile Modulus v (GPa) | 3.4 | 1.9 | 2.2 | 3 | N/A |
| v/f | 0.87 | 1.00 | 0.96 | 0.96 | N/A |
| CTE f (10⁻⁵/degree C) | 5.4 | 8.8 | 7.1 | 6.1 | N/A |
| CTE v (10⁻⁵/degree C) | 5.0 | 8.8 | 7.5 | 6.6 | N/A |
| f/v | 0.93 | 1.00 | 1.06 | 1.08 | N/A |
| Notched Izod Impact strength f (J/m) | 120 | 450 | 132 | 146 | N/A |
| Notched Izod Impact strength v (J/m) | 103 | 450 | 143 | 130 | N/A |
| v/f | 0.86 | 1.00 | 1.08 | 0.89 | N/A |
| Appearance of molded plaque surface | Pearlescent yellow | Pearlescent yellow | Pearlescent yellow | Pearlescent yellow | N/A |

**Table 1 (part 3)**

| | Comparative Example 5 | Comparative Example 6 | Comparative Example 7 | Comparative Example 8 |
|---|---|---|---|---|
| Filler | HR-90 | MICA-100 | PK-P | Nygloss 8 |
| Type of filler | Phlogopite mica | Muscovite mica | Talc | Wollastonite |
| Average particle size of filler (micro m) | 720 | 75 | 6.5 | 136 |
| Average aspect ratio of filler | | 35 | | |
| Bulk density of filler (g/ml) | 0.56 | 0.26 | 0.42 | 0.3 |
| Viscosity of C Component (Pa·s) | 0.5 | 0.22 | 1.4 | 2.2 |
| Lump or segmentation of filler in C Component (Yes/No) | Yes | No | No | Yes |
| Content of filler in molded plaques (wt%) | 15 | 5 | 15 | 15 |
| Flexural Modulus f (GPa) | N/A | 2.52 | 2.69 | 3.34 |
| Flexural Modulus v (GPa) | N/A | 2.6 | 2.72 | 2.44 |
| v/f | N/A | 1.03 | 1.01 | 0.73 |
| Tensile Modulus f (GPa) | N/A | 2.37 | 2.52 | 3.2 |
| Tensile Modulus v (GPa) | N/A | 2.35 | 2.45 | 2.61 |
| v/f | N/A | 0.99 | 0.97 | 0.82 |
| CTE f (10⁻⁵/degree C) | N/A | 6.78 | 6.81 | 7.62 |
| CTE v (10⁻⁵/degree C) | N/A | 6.71 | 6.36 | 4.74 |
| f/v | N/A | 1.01 | 0.93 | 0.62 |
| Notched Izod Impact strength f (J/m) | N/A | 160 | 150 | 57 |
| Notched Izod Impact strength v (J/m) | N/A | 176 | 147 | 48 |
| v/f | N/A | 1.10 | 0.98 | 0.84 |
| Appearance or molded plaque surface | N/A | Pearlescent yellow | Solid light yellow | Solid light yellow |

## Claims

1. A liquid molding resin component for use in a reaction injection molding process, the liquid molding resin component comprising:
a liquid resin reaction monomer comprising metathesis polymerizable cycloolefin; and
nonswelling mica,
wherein the nonswelling mica have an average particle size in a range of 35 micro m to 500 micro m and have a bulk density in a range of 0.10 g/ml to 0.27 g/ml.

2. A liquid molding resin component according to Claim 1, wherein the nonswelling mica have an average particle size in a range of 40 micro m to 200 micro m and may have an average aspect ratio in a range of 10 to 200.

3. A liquid molding resin component according to Claim 1, further comprising one of (i) an activator component of a metathesis polymerization catalyst system, and (ii) a catalyst component of a metathesis polymerization catalyst system, wherein the activator component may include an alkyl aluminum compound and the catalyst component may include at least one of a tungsten compound and a molybdenum compound.

4. A liquid molding resin component according to Claim 1, wherein the nonswelling mica are all the same type of nonswelling mica.

5. A liquid molding resin component according to Claim 1, wherein the nonswelling mica have a bulk density in a range of 0.14 g/ml to 0.25 g/ml, and may have an average particle size in a range of 40 micro m to 300 micro m and have an average aspect ratio in a range of 20 to 120.

6. A liquid molding resin component according to Claim 1 or Claim 5, wherein the nonswelling mica comprises at least one of Muscovite and Phlogopite.

7. A liquid resin component system for use in a reaction injection molding process, said component system comprising a plurality of liquid resin components, wherein each of the liquid resin components includes a reaction monomer comprising metathesis polymerizable cycloolefin, wherein at least one of the liquid resin components includes a catalyst component of a metathesis polymerization catalyst system and at least one of the liquid resin components includes an activator component of a metathesis polymerization catalyst system, and wherein at least one of the liquid resin components includes nonswelling mica having an average particle size in a range of 35 micro m to 500 micro m and having a bulk density in a range of 0.10 g/ml to 0.27 g/ml.

8. A liquid resin component system according to Claim 7, wherein the nonswelling mica have an average aspect ratio in a range of 10 to 200 and wherein the nonswelling mica may be present in an amount such that the nonswelling mica constitute from 6 to 50 parts by weight of the total liquid resin.

9. A liquid resin component system according to Claim 7, wherein the activator includes an alkylaluminum compound and the catalyst includes at least one of a tungsten compound and a molybdenum compound.

10. A method of making a liquid resin component for use in an injection molding process, said method comprising the steps of:
providing a liquid resin reaction monomer wherein the reaction monomer is metathesis polymerizable cycloolefin; and
adding nonswelling mica to the liquid resin reaction monomer, wherein the nonswelling mica have an average particle size in a range of 35 micro m to 500 micro m and have a bulk density in a range of 0.10 g/ml to 0.27 g/ml.

11. A method according to Claim 10, wherein the nonswelling mica have an average aspect ratio in a range of 10 to 200 and wherein the nonswelling mica may be added in an amount such that the nonswelling mica constitute from 6 to 50 parts by weight of the total liquid resin.

12. A method according to Claim 11, wherein the nonswelling mica comprises at least one of Muscovite and Phlogopite.

13. A method of making a molded resin product, comprising the steps of:
providing a plurality of liquid resin components that each include a reaction monomer that is metathesis polymerizable cycloolefin, wherein the reaction monomer in each of the plurality of liquid resin components is the same reaction monomer, wherein at least one of the liquid resin components includes a metathesis catalyst but not an activator, at least one of the liquid resin components includes a metathesis activator but not a metathesis catalyst, and at least one of the liquid resin components includes nonswelling mica;
mixing the liquid resin components; and
injecting the mixed liquid resin components into a mold at a predetermined temperature,
wherein the nonswelling mica have an average particle size in a range of 35 micro m to 500 micro m and have a bulk density in a range of 0.10 g/ml to 0.27 g/ml.

14. A method according to Claim 13, wherein the reaction monomer is metathesis polymerizable cycloolefin, and the nonswelling mica have an average aspect ratio in a range of 10 to 200 and wherein the nonswelling mica may be present in an amount such that the nonswelling mica constitute from 6 to 50 parts by weight of the total liquid resin.

15. A method according to Claim 14, wherein the nonswelling mica comprises at least one of Muscovite and Phlogopite.

## Patentansprüche

1. Flüssigformharzkomponente zur Verwendung in einem Reaktionsspritzgießverfahren, wobei die Flüssigformharzkomponente aufweist:
ein Flüssigharz-Reaktionsmonomer, das durch Metathese polymerisierbares Cycloolefin aufweist;
und
nicht-quellenden Glimmer,
wobei der nicht-quellende Glimmer eine durchschnittliche Partikelgröße in einem Bereich von 35 Mikrometer bis 500 Mikrometer hat und eine Schüttdichte in einem Bereich von 0,10 g/ml bis 0,27 g/ml hat.

2. Flüssigformharzkomponente nach Anspruch 1, wobei der nicht-quellende Glimmer eine durchschnittliche Partikelgröße in einem Bereich von 40 Mikrometer bis 200 Mikrometer hat und ein durchschnittliches Aspektverhältnis in einem Bereich von 10 bis 200 haben kann.

3. Flüssigformharzkomponente nach Anspruch 1, ferner aufweisend eine von (i) einer Aktivatorkomponente eines Katalysatorsystems für eine Metathese-Polymerisation und (ii) einer Katalysatorkomponente eines Katalysatorsystems für eine Metathese-Polymerisation, wobei die Aktivatorkomponente eine Alkylaluminiumverbindung enthalten kann und die Katalysatorkomponente mindestens eine von einer Wolframverbindung und einer Molybdänverbindung enthalten kann.

4. Flüssigformharzkomponente nach Anspruch 1, wobei der gesamte nicht-quellende Glimmer von derselben Art nicht-quellenden Glimmers ist.

5. Flüssigformharzkomponente nach Anspruch 1, wobei der nicht-quellende Glimmer eine Schüttdichte in einem Bereich von 0,14 g/ml bis 0,25 g/ml hat und eine durchschnittliche Partikelgröße in einem Bereich von 40 Mikrometer bis 300 Mikrometer haben kann und ein durchschnittliches Aspektverhältnis in einem Bereich von 20 bis 120 hat.

6. Flüssigformharzkomponente nach Anspruch 1 oder Anspruch 5, wobei der nicht-quellende Glimmer mindestens einen von Muskovit und Phlogopit aufweist.

7. Flüssigharzkomponentensystem zur Verwendung in einem Reaktionsspritzgießverfahren, wobei das Komponentensystem eine Mehrzahl von Flüssigharzkomponenten aufweist, wobei jede der Flüssigharzkomponenten ein Reaktionsmonomer enthält, das durch Metathese polymerisierbares Cycloolefin aufweist, wobei mindestens eine der Flüssigharzkomponenten eine Katalysatorkomponente eines Katalysatorsystems für eine Metathese-Polymerisation enthält und mindestens eine der Flüssigharzkomponenten eine Aktivatorkomponente eines Katalysatorsystems für eine Metathese-Polymerisation enthält, und wobei mindestens eine der Flüssigharzkomponenten nicht-quellenden Glimmer aufweist, die eine durchschnittliche Partikelgröße in einem Bereich von 35 Mikrometer bis 500 Mikrometer hat und eine Schüttdichte in einem Bereich von 0,10 g/ml bis 0,27 g/ml hat.

8. Flüssigharzkomponentensystem nach Anspruch 7, wobei der nicht-quellende Glimmer ein durchschnittliches Aspektverhältnis in einem Bereich von 10 bis 200 hat, und wobei der nicht-quellende Glimmer in einer solchen Menge vorhanden sein kann, dass der nicht-quellende Glimmer 6 bis 50 Gewichtsanteile des gesamten Flüssigharzes ausmacht.

9. Flüssigharzkomponentensystem nach Anspruch 7, wobei der Aktivator eine Alkylaluminiumverbindung enthält und der Katalysator mindestens eine von einer Wolframverbindung und einer Molybdänverbindung enthält.

10. Verfahren zum Herstellen einer Flüssigharzkomponente zur Verwendung in einem Spritzgießverfahren, wobei das Verfahren die folgenden Schritte aufweist:
Bereitstellen eines Flüsssigharz-Reaktionsmonomers, wobei das Reaktionsmonomer durch Metathese polymerisierbares Cycloolefin ist; und
Zugeben von nicht-quellenden Glimmer zum Flüssigharz-Reaktionsmonomer, wobei der nicht-quellende Glimmer eine durchschnittliche Partikelgröße in einem Bereich von 35 Mikrometer bis 500 Mikrometer hat und eine Schüttdichte in einem Bereich von 0,10 g/ml bis 0,27 g/ml hat.

11. Verfahren nach Anspruch 10, wobei der nicht-quellende Glimmer ein durchschnittliches Aspektverhältnis in einem Bereich von 10 bis 200 hat, und wobei der nicht-quellende Glimmer in einer solchen Menge zugegeben werden kann, dass der nicht-quellende Glimmer 6 bis 50 Gewichtsanteile des gesamten Flüssigharzes ausmacht.

12. Verfahren nach Anspruch 11, wobei der nicht-quellende Glimmer mindestens einen von Muskovit und Phlogopit aufweist.

13. Verfahren zum Herstellen eines gegossenen Harzprodukts, aufweisend die folgenden Schritte:
Bereitstellen einer Mehrzahl von Flüssigharzkomponenten, von denen jede ein Reaktionsmonomer enthält, das durch Metathese polymerisierbares Cycloolefin ist, wobei das Reaktionsmonomer in jeder der Mehrzahl von Flüssigharzkomponenten das gleiche Reaktionsmonomer ist, wobei mindestens eine der Flüssigharzkomponenten einen Metathese-Katalysator aber keinen Aktivator enthält, mindestens eine der Flüssigharzkomponenten einen Metathese-Aktivator aber keinen Metathese-Katalysator enthält und mindestens eine der Flüssigharzkomponenten nicht-quellenden Glimmer enthält;
Mischen der Flüssigharzkomponenten; und
Einspritzen der gemischten Flüssigharzkomponenten in eine Gussform bei einer vorbestimmten Temperatur,
wobei der nicht-quellende Glimmer eine durchschnittliche Partikelgröße in einem Bereich von 35 Mikrometer bis 500 Mikrometer hat und eine Schüttdichte in einem Bereich von 0,10 g/ml bis 0,27 g/ml hat.

14. Verfahren nach Anspruch 13, wobei das Reaktionsmonomer durch Metathese polymerisierbares Cycloolefin ist und der nicht-quellende Glimmer ein durchschnittliches Aspektverhältnis in einem Bereich von 10 bis 200 hat, und wobei der nicht-quellende Glimmer in einer solchen Menge vorhanden sein kann, dass der nicht-quellende Glimmer 6 bis 50 Gewichtsanteile des gesamten Flüssigharzes ausmacht.

15. Verfahren nach Anspruch 14, wobei der nicht-quellende Glimmer mindestens einen von Muskovit und Phlogopit aufweist.

## Revendications

1. Composant pour résine de moulage liquide destiné à être utilisé dans un procédé de moulage par injection réactionnel, le composant pour résine de moulage liquide comprenant :
un monomère réactionnel de résine liquide comprenant une cyclooléfine polymérisable par métathèse ; et
un mica non-gonflant,
le mica non-gonflant ayant une granulométrie moyenne entre 35 µm et 500 µm et une densité apparente entre 0,10 g/ml et 0,27 g/ml.

2. Composant pour résine de moulage liquide selon la revendication 1, dans lequel le mica non-gonflant a une granulométrie moyenne entre 40 µm et 200 µm et peut avoir un rapport d'aspect moyen entre 10 et 200.

3. Composant pour résine de moulage liquide selon la revendication 1, comprenant en outre un parmi (i) un composant activateur d'un système catalytique de polymérisation par métathèse, et (ii) un catalyseur composant d'un système catalytique de polymérisation par métathèse, le composant activateur pouvant inclure un composé alkylaluminium et le composant catalytique pouvant inclure au moins parmi un composé du tungstène et un composé du molybdène.

4. Composant pour résine de moulage liquide selon la revendication 1, dans lequel le mica non-gonflant est un ensemble de micas non-gonflants tous du même type.

5. Composant pour résine de moulage liquide selon la revendication 1, dans lequel les micas non-gonflants ont une densité apparente entre 0,14 g/ml et 0,25 g/ml et peuvent avoir une granulométrie moyenne entre 40 µm et 300 µm et un rapport d'aspect moyen entre 20 et 120.

6. Composant pour résine de moulage liquide selon la revendication 1 ou 5, dans lequel le mica non-gonflant comprend au moins un parmi la muscovite et la phlogopite.

7. Système de composants de résine liquide destiné à être utilisé dans un procédé de moulage par injection réactionnel, ledit système de composants comprenant plusieurs composants de résine liquide, chacun des composants de résine liquide incluant un monomère réactionnel comprenant une cyclooléfine polymérisable par métathèse, où au moins un des composants de résine liquide inclut un composant catalytique d'un système catalytique de polymérisation par métathèse et au moins un parmi les composants de résine liquide incluant un composant activateur d'un système catalytique de polymérisation par métathèse, et où au moins un des composants de résine liquide inclut un mica non-gonflant ayant une granulométrie moyenne entre 35 µm et 500 µm et une densité apparente entre 0,10 g/ml et 0,27 g/ml.

8. Composant pour résine liquide selon la revendication 7, dans lequel les micas non-gonflants ont un rapport d'aspect moyen entre 10 et 200 et dans lequel les micas non-gonflants peuvent être présents à une hauteur telle que les micas non-gonflants constituent 6 à 50 parties en poids du total de la résine liquide.

9. Système de composants de résine liquide selon la revendication 7, dans lequel l'activateur inclut un composé alkylaluminium et le catalyseur contient au moins un composé du tungstène et un composé du molybdène.

10. Procédé de fabrication d'un composant de résine liquide destinée à être utilisée dans un procédé de moulage par injection, ledit procédé comprenant les étapes de :
fabrication d'un, le monomère réactionnel étant une cyclooléfine monomère réactionnel de résine liquide polymérisable par métathèse ; et
ajout de micas non-gonflants au monomère réactionnel de résine liquide ayant une granulométrie moyenne entre 35 µm et 500 µm et une densité apparente entre 0,10 g/ml et 0,27 g/ml.

11. Procédé selon la revendication 10, dans lequel les micas non-gonflants ont un rapport d'aspect moyen entre 10 et 200 et dans lequel on peut ajouter les micas non-gonflants à une hauteur telle que les micas non-gonflants constituent 6 à 50 parties en poids du total de la résine liquide.

12. Procédé selon la revendication 11, dans lequel le mica non-gonflant contient au moins un parmi la muscovite et la phlogopite.

13. Procédé de fabrication d'un produit en résine moulée, comprenant les étapes de :
création de plusieurs composants de résine liquide qui incluent chacun un monomère réactionnel qui est une cyclooléfine polymérisable par métathèse, le monomère réactionnel dans chacun des composants de résine liquide étant le même monomère réactionnel, au moins un des composants de résine liquide incluant un catalyseur de métathèse mais pas un activateur, au moins un des composants de résine liquide incluant un activateur de métathèse mais pas un catalyseur de métathèse, et au moins un des composants de résine liquide incluant un mica non-gonflant ;
mélange des composants de résine liquide ; et
injection des composants de résine liquide mélangés dans un moule à une température prédéterminée,
où les micas non-gonflants ont une granulométrie moyenne entre 35 µm et 500 µm et une densité apparente entre 0,10 g/ml et 0,27 g/ml.

14. Procédé selon la revendication 13, dans lequel le monomère réactionnel est une cyclooléfine polymérisable par métathèse, et les micas non-gonflants ont un rapport d'aspect moyen entre 10 et 200, et où les micas non-gonflants peuvent être présents à une hauteur telle que les micas non-gonflants constituent 6 à 50 parties en poids du total de la résine liquide.

15. Procédé selon la revendication 14, dans lequel le mica non-gonflant comprend au moins un entre la muscovite et la phlogopite.
